# EUROPÄISCHE PATENTSCHRIFT

(11) **EP 1 350 477 B1**
(45) Veröffentlichungstag und Bekanntmachung des Hinweises auf die Patenterteilung: **16.01.2008**
(21) Anmeldenummer: 03003353.4
(22) Anmeldetag: 13.02.2003
(51) Int. Cl.: A61B 17/34

(54) **Trokarhülse**
Trocar sleeve
Manchon de trocart

(30) Priorität: 02.04.2002 DE 10214551
(43) Veröffentlichungstag der Anmeldung: 08.10.2003
(73) Patentinhaber: Pajunk GmbH & Co. KG Besitzverwaltung, 78187 Geisingen (DE)
(72) Erfinder: Pajunk, Horst, 78187 Geisingen (DE); Pajunk, Heinrich, 78187 Geisingen-Kirchen-Hausen (DE)
(74) Vertreter: Manitz, Finsterwald & Partner GbR

(56) Entgegenhaltungen:
- EP-A- 0 513 962
- EP-A- 0 517 248
- EP-A- 0 564 373
- EP-A- 0 873 721
- US-A- 5 409 464
- US-A- 5 993 471

## Beschreibung

Die vorliegende Erfindung betrifft eine Trokarhülse gemäß dem Oberbegriff des Anspruchs 1.

Um bei der Laparoskopie sowie der minimal invasiven Chirurgie die benötigten Instrumente in das Körperinnere, z. B. die Bauchhöhle einbringen zu können, wird üblicherweise die erforderliche Öffnung in der Bauchdecke durch einen Trokar erzeugt. Auf dem Trokar sitzt eine Trokarhülse, die nach Entfernen des Trokars den Zugangskanal für die verwendeten Instrumente bildet. Um Raum für die Beobachtung bzw. den operativen Eingriff zu schaffen, wird insbesondere bei der minimal invasiven Chirurgie des Bauchraumes Gas unter Druck in die Bauchhöhle eingebracht, um eine Anhebung der Bauchdecke zu erreichen. Damit der auf diese Weise aufgebaute Überdruck aufrechterhalten bleibt, besitzen die Trokarhülsen üblicherweise ein Ventil, das beim Einführen eines Instruments geöffnet wird und in Abwesenheit eines Instruments die Trokarhülse verschließt.

Trokarhülsen der eingangs genannten Art sind in vielfältiger Form bekannt. Problematisch ist jedoch bei den meisten bekannten Trokarhülsen, dass keine ausreichende Dichtigkeit des Ventils gewährleistet ist, insbesondere dass keine ausreichende automatische Schließung des Ventils erfolgt, nachdem ein eingesetztes Instrument aus der Trokarhülse wieder herausgezogen wird. Um die erforderliche Dichtigkeit gewährleisten zu können, werden die Dichtlippen bei bekannten Ventilanordnungen oftmals mit relativ hoher Kraft aneinander gepresst. Dies hat jedoch den Nachteil, dass auf ein in das Körperinnere eingeführtes Instrument eine hohe Klemmkraft wirkt, worunter das Gefühl für feinmechanische Bewegungen des behandelnden Chirurgen leidet. Außerdem ist beim Einführen des Instruments eine relativ hohe Kraft zu überwinden.

Aus der US 5,993,471 sowie der EP 0 513 962 sind jeweils Trokarhülsen der eingangs genannten Art bekannt. Diese bekannten Trokarhülsen umfassen jeweils Ventileinsätze, deren distaler Bereich durch eine schlitzförmige Öffnung in zwei Ventilklappen aufgeteilt wird. Durch die Aufteilung in lediglich zwei Ventilklappen entsteht ein relativ hoher Klemmeffekt, so dass die Handhabbarkeit der eingeführten Instrumente eingeschränkt ist und beim Zurückziehen der Instrumente ein Verhaken der Instrumente erfolgen kann.

Aus der EP 0 517 248 ist eine Trokarhülse mit einem kuppelförmigen, eingangsseitig angeordneten Abdichtelement bekannt, das eine konzentrisch angeordnete Öffnung für das einzuführende Instrument besitzt. An dieses Abdichtelement schließt sich eine entenschnabelförmig ausgebildete weitere Dichtung mit einer distal angeordneten schlitzförmigen Öffnung an, die beim Einführen des Instruments aufgeweitet wird.

Auch die EP 0 873 721 zeigt ein entsprechendes Ventilelement mit schlitzförmiger Öffnung, die beim Einführen des Instruments aufgeweitet wird, wobei diese schlitzförmige Öffnung S-förmig ausgebildet ist.

Es ist eine Aufgabe der vorliegenden Erfindung, eine Trokarhülse der eingangs genannten Art so auszubilden, dass eine sichere Abdichtung der Trokarhülse auch nach Entfernen eines zuvor eingeführten Instrumentes gewährleistet ist. Weiterhin soll die auf ein eingeführtes Instrument wirkende bzw. beim Einführen eines Instrumentes wirkende Klemmkraft reduziert werden.

Ausgehend von einer Trokarhülse der eingangs genannten Art wird diese Aufgabe erfindungsgemäß - durch die Merkmale des Anspruchs 1 gelöst.

Durch die spezielle kuppelförmige Form des Ventils wird eine besonders sichere Abdichtung der Trokarhülse erzielt. Durch die kuppelförmige Ausgestaltung ist gewährleistet, dass die durch das elastische Ventilmate-rial erzeugten Rückstellkräfte in den Dichtlippen tangential verlaufen und somit einen maximalen Anpressdruck der Dichtlippen aneinander bewirken. Dieser Anpressdruck wird noch dadurch erhöht, dass an der Außenseite des kuppelförmigen Wandabschnittes zumindest ein taschenförmiger Bereich ausgebildet ist, in dem der beispielsweise innerhalb der Bauchhöhle erzeugte Überdruck aufgenommen wird, so dass wiederum ein Druck auf die Außenfläche des kuppelförmigen Wandabschnittes erzeugt wird. Auch dieser Druck wird durch die kuppelförmige Form in optimaler Weise in einen gegenseitigen Anpressdruck der Dichtlippen in tangentialer Richtung umgesetzt. Vorteilhaft umgibt der taschenförmige Bereich dabei den kuppelförmigen Wandabschnitt ringförmig. Grundsätzlich ist auch eine Aufteilung in mehrere taschenförmige Bereiche, beispielsweise durch Einfügen von Zwischenwänden möglich.

Der maximale Anpressdruck der Dichtlippen ist nur bei geschlossenem Ventil vorhanden, da nur in diesem Zustand die auf die Dichtlippen wirkenden Schließkräfte jeweils tangential zur kuppelförmigen Oberfläche des Ventilabschnittes wirken. Daher sind die Klemmkräfte der Dichtlippen bei eingeführtem Instrument deutlich geringer, da in diesem Fall die durch die Rückstellkräfte erzeugten Kraftkomponenten nicht genau gegensinnig wirken, sondern ebenfalls Kraftkomponenten vorhanden sind, die beispielsweise in proximaler Richtung wirken. Somit sind die auf die Dichtlippen wirkenden Klemmkräfte bei einem eingeführten Instrument verringert, wodurch sowohl das Einführen des Instrumentes erleichtert als auch die feinfühlige Bedienbarkeit eines eingeführten Instrumentes verbessert wird.

Nach einer vorteilhaften Ausführungsform der Erfindung besitzt der kuppelförmige Wandabschnitt die Form einer Kugelkalotte. Grundsätzlich kann der kuppelförmige Wandabschnitt auch eine von der Kugelkalottenform abweichende Form, beispielsweise die Form eines Rotationsparaboluids oder eine ähnliche gekrümmte Form besitzen.

Nach einer weiteren bevorzugten Ausführungsform der Erfindung ist zumindest der den Einführbereich bildende kuppelförmige Wandabschnitt - aus elastischem Material, insbesondere aus einem weichelastischen Kunststoff, ausgebildet. Die schlitzförmigen Öffnungen schneiden sich im distalen Endpunkt des kuppelförmigen Wandabschnitts, wobei die schlitzförmigen Öffnungen vorteilhaft in einer Draufsicht auf den kuppelförmigen Wandabschnitt Geraden bilden.

Weiterhin ist der kuppelförmige Wandabschnitt durch die schlitzförmigen Öffnungen in im Wesentlichen gleich große, symmetrisch zum Schnittpunkt der schlitzförmigen Öffnungen angeordnete Ventilklappen aufgeteilt. Durch diese Ausgestaltungen wird eine besonders hohe Dichtigkeit der erfindungsgemäßen Trokarhülse erreicht.

Der kuppelförmige Wandabschnitt umfasst drei oder vier Ventilklappen. Insbesondere die Ausbildung mit vier Ventilklappen ist vorteilhaft, da aufgrund der größeren Anzahl von Ventilklappen die Beweglichkeit dieser Ventilklappen erhöht und damit die Bedienbarkeit eines eingesetzten Instrumentes verbessert wird.

Erfindungsgemäß ist jede Ventilklappe mit zumindest einem Verstärkungselement versehen. Dabei sind diese Verstärkungselemente als Verstärkungsstege ausgebildet. Durch das Anbringen bzw. das Vorsehen von Verstärkungsstegen wird die Form der Ventilklappen stabilisiert und deren Federkraft erhöht, so dass die Dichtigkeit einer erfindungsgemäßen Trokarhülse weiter verbessert wird.

Erfindungsgemäß verlaufen die Verstärkungsstege jeweils ausgehend von dem proximalen Ende der Ventilklappe in Richtung des distalen Endes der Ventilklappe, wodurch eine zusätzliche Kraft in Schließrichtung des Ventils erzeugt wird.

Dabei sind die Verstärkungsstege sich in distaler Richtung verjüngend ausgebildet Durch die verjüngende Ausbildung wird zum einen in den Basisabschnitten der Ventilklappen eine höhere Rückstellkraft erzeugt, wobei gleichzeitig aufgrund der verjüngten Enden im Bereich der innen liegenden Spitzen der Ventilklappen die Klemmkraft etwas reduziert wird, um damit das Einführen eines Instrumentes zu erleichtern bzw. die Bedienbarkeit und Feinfühligkeit für ein eingesetztes Instrument zu verbessern. Grundsätzlich ist es auch möglich, dass an jeder Ventilklappe mehrere Verstärkungselemente vorgesehen sind.

Nach einer weiteren bevorzugten Ausführungsform der Erfindung ist das Verstärkungselement einstückig mit der Ventilklappe ausgebildet und insbesondere an dieser jeweils angeformt. Vorteilhaft ist dabei das Verstärkungselement aus dem gleichen Material wie die Ventilklappe oder aus einem anderen Material als die Ventilklappe, jedoch ebenfalls elastisch, ausgebildet. Ist das Verstärkungselement aus dem gleichen Material und insbesondere einstückig mit der Ventilklappe ausgebildet, so kann das gesamte Ventil in einem einfachen Spritzgussverfahren hergestellt werden.

Nach einer weiteren vorteilhaften Ausführungsform der Erfindung ist das Verstärkungselement jeweils an der innen liegenden Seite der Ventilklappe vorgesehen. Die innen liegende Anordnung hat den Vorteil, dass beim Einführen eines insbesondere einen kreisförmigen Querschnitt aufweisenden Instrumentes dieses beim Einsetzen in den Einführbereich praktisch ausschließlich an den Verstärkungselementen, nicht jedoch an den Innenseiten der Ventilklappen selbst zur Anlage kommt. Somit wird die Kontaktfläche zwischen Ventil und Instrument beim Einführen und beim Handhaben des Instrumentes deutlich verringert, so dass auch die Reibungskraft, die zwischen Ventil und Instrument wirkt, verringert wird. Dadurch ist sowohl ein deutlich leichteres Einführen des Instrumentes als auch eine bessere Handhabbarkeit der eingesetzten Instrumente möglich.

Nach einer weiteren vorteilhaften Ausführungsform der Erfindung ist am proximalen Ende des Einführbereichs ein insbesondere ringförmiger Basisabschnitt des Ventils ausgebildet. Dieser ringförmige Basisabschnitt kann beispielsweise einen sich in distaler Richtung erstreckenden ringförmigen Flansch umfassen. Über den ringförmigen Basisabschnitt und insbesondere den ringförmigen Flansch ist eine Fixierung des Ventils innerhalb des Ventilgehäuses möglich, wodurch verhindert wird, dass das Ventil beim Einsetzen eines Instrumentes bzw. beim Entnehmen eines Instrumentes aus dem Ventilgehäuse und damit aus der Trokarhülse austreten kann.

Nach einer weiteren vorteilhaften Ausführungsform der Erfindung ist die Begrenzungswand als Teil des Ventilgehäuses oder als separates Teil ausgebildet. Grundsätzlich kann die Begrenzungswand jedoch auch als Teil des Ventils ausgebildet sein. Wesentlich ist, dass der zwischen der Begrenzungswand und der Außenseite des kuppelförmigen Wandabschnitts gebildete taschenförmige Bereich in proximaler Richtung luftdicht abgeschlossen ist, damit in diesem taschenförmigen Bereich ein Druck aufgebaut wird, der die Schließkraft des Ventils und damit den Anpressdruck der Dichtlippen aneinander in geschlossenem Zustand des Ventils erhöht.

Weitere vorteilhafte Ausführungsformen der Erfindung sind in den Unteransprüchen angegeben.

Die Erfindung wird nachfolgend anhand eines Ausführungsbeispiels unter Bezugnahme auf die Zeichnung näher erläutert;

in dieser zeigen:
- Figur 1: eine Seitenansicht einer erfindungsgemäß ausgebildeten Trokarhülse,
- Figur 2: eine vereinfachte perspektivische Ansicht eines erfindungsgemäß ausgebildeten Ventils in geschlossenem Zustand,
- Figur 3: das Ventil nach Figur 2 mit eingeführtem Instrument,
- Figur 4: eine Draufsicht gemäß dem Pfeil A aus Figur 3,
- Figur 5: eine Draufsicht auf das Ventil aus Figur 2,
- Figur 6: einen Querschnitt entlang der Linien A - A aus Figur 5,
- Figur 7: eine Unteransicht des Ventils aus Figur 2 und
- Figur 8: eine Schnittansicht entlang der Linie B - B aus Figur 7.

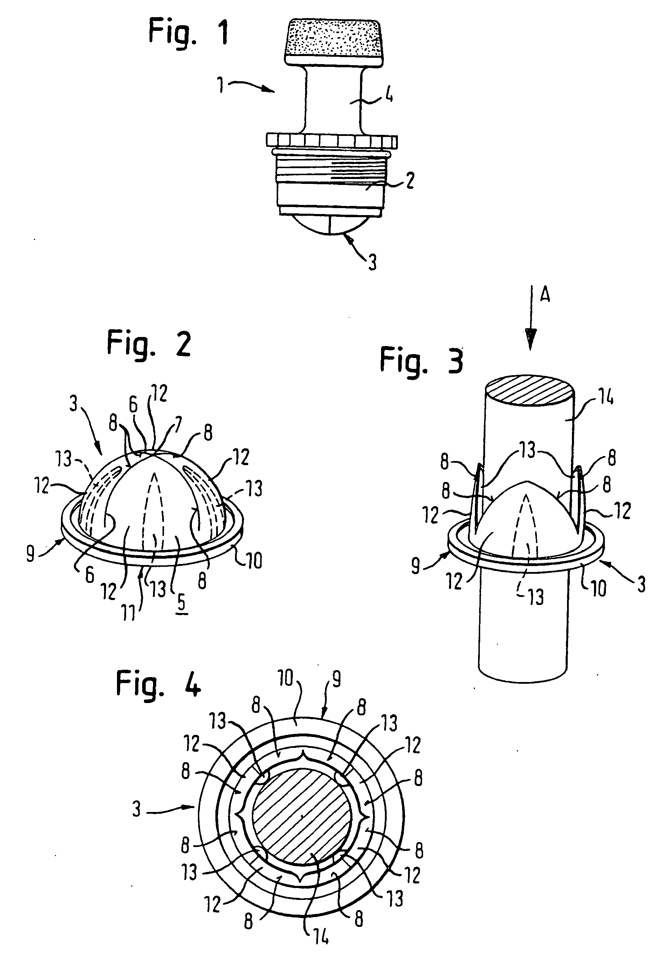

Figur 1 zeigt eine Trokarhülse 1, deren distales Ende als Ventilgehäuse ausgebildet ist. In das offene Ende des Ventilgehäuses 2 ist ein Ventil 3 so eingesetzt, dass lediglich das distale Ende des Ventils 3 an der Unterseite des Ventilgehäuses 2 herausragt.

Der Mittelbereich der Trokarhülse 1 ist als Einführkanal ausgebildet, wobei an dem einführseitig gelegenen, proximalen Ende der Trokarhülse 1 eine aus elastischem Kunststoffmaterial ausgebildete Abdichtkappe vorgesehen ist. Diese Abdichtkappe besitzt an ihrer Oberseite eine in der Figur 1 nicht sichtbare, konzentrisch angeordnete Öffnung, durch die ein Instrument in den Einführkanal einführbar ist. Durch die elastische Abdichtkappe wird dabei die Außenseite des Instrumentes umschlossen und somit abgedichtet, auch wenn das Ventil 3 geöffnet ist. Der Einführkanal ist mit dem Bezugszeichen 4 versehen.

Figur 2 zeigt den kuppelförmigen Grundaufbau des Ventils 3 in perspektivischer Darstellung. Das Ventil 3 umfasst einen kuppelförmigen Wandabschnitt 5, in dem schlitzförmige Öffnungen 6 ausgebildet sind, die von dem distalen Scheitelpunkt 7 des kuppelförmigen Wandabschnitts 5 in proximaler Richtung verlaufen. Durch die schlitzförmigen Öffnungen 6 werden Dichtlippen 8 gebildet, die in geschlossenem Zustand des Ventils 3, wie er in Figur 2 gezeigt ist, aufgrund der Elastizität des Ventilmaterials unter Anpressdruck aneinander anliegen.

Am proximalen Ende des Ventils 3 ist ein ringförmiger Basisabschnitt 9 ausgebildet, der einen ebenfalls ringförmig ausgebildeten, sich in distaler Richtung erstreckenden Flansch 10 umfasst.

Der kuppelförmige Wandabschnitt 5 bildet einen Einführbereich 11 für ein in das Körperinnere einzuführende Instrument, wobei aufgrund der kuppelförmigen Ausbildung sich der Einführbereich 11 in distaler Richtung bis zu dem distalen Scheitelpunkt 7 verengt.

Das zwischen den Dichtlippen gelegene Ventilmaterial bildet Ventilklappen 12, an deren Innenseite jeweils sich von dem Basisabschnitt 9 zum distalen Scheitelpunkt 7 hin erstreckende Verstärkungsstege 13 vorgesehen sind, die in Figur 2 gestrichelt dargestellt sind. Diese Verstärkungsstege 13 laufen in distaler Richtung konisch verjüngt zu und bewirken eine Verstärkung der Ventilklappen 12 und damit einen erhöhten Anpressdruck der Dichtlippen gegeneinander.

Figur 3 zeigt das Ventil gemäß Figur 2 mit einem eingeführten Instrument 14. Aus Figur 3 ist zu erkennen, dass die Ventilklappen 12 nach oben zurückgeklappt sind und lediglich mit den Verstärkungsstegen 13 an der Außenseite des Instrumentes 14 anliegen. Somit besteht zwischen den Ventilklappen 12 und dem Instrument 14 im besten Fall lediglich ein linienförmiger Kontakt, so dass die Reibung zwischen dem Instrument 14 und den Ventilklappen 12 deutlich verringert ist.

Dies ist auch aus der in Figur 4 dargestellten Draufsicht gemäß dem Pfeil A aus Figur 3 deutlich zu erkennen. Zwischen dem Ventil 14 und den Ventilklappen 12 bestehen lediglich vier linienförmige Kontaktbereiche, so dass sowohl das Einführen des Instrumentes 14 als auch die Bewegung des eingeführten Instrumentes 14 mit sehr geringem Kraftaufwand möglich ist.

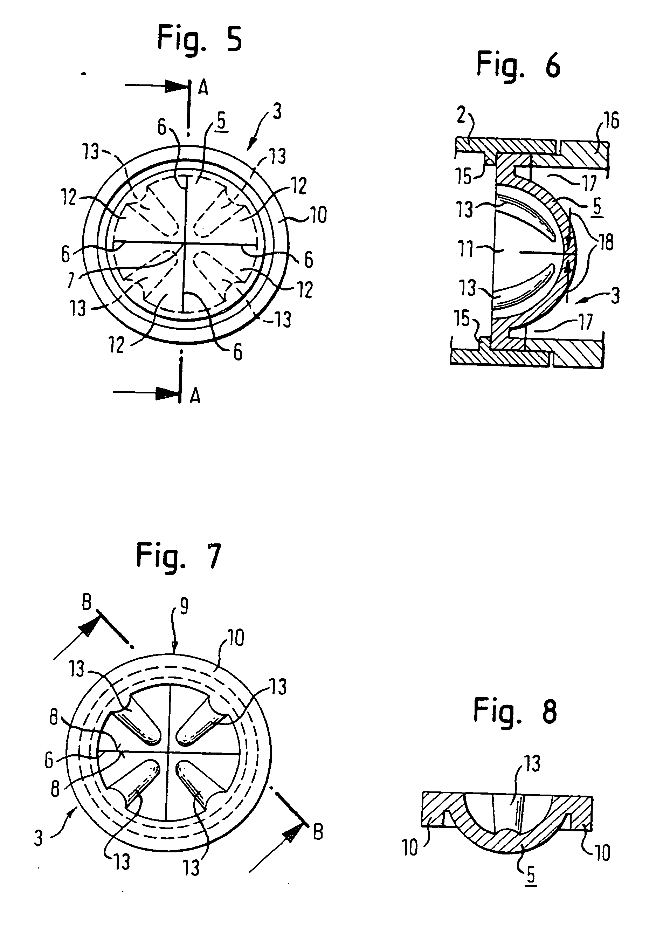

Die Anordnung der Verstärkungsstege 13 ist auch nochmals der Figur 5 näher zu entnehmen. Insbesondere ist hier die konische Form der Verstärkungsstege 13 sowie deren Verlauf von dem Basisabschnitt 10 in Richtung des distalen Scheitelpunktes 7 ersichtlich.

In der Schnittansicht gemäß Figur 6 ist die Anordnung des Ventils 3 innerhalb des Ventilgehäuses 2 dargestellt. Dabei ist zu erkennen, dass eine Verschiebung des Ventils 3 durch an der Innenseite des Ventilgehäuses 2 vorgesehene Ansätze 15 sowie eine in das Ventilgehäuse eingesetzte zylinderförmige Begrenzungswand 16 verhindert wird.

Zwischen der Innenseite der Begrenzungswand 16 und der Außenseite des kuppelförmigen Wandabschnittes 5 ist ein taschenförmiger Bereich 17 ausgebildet, der den kuppelförmigen Wandabschnitt 5 ringförmig umgibt. Bei eingesetzter Trokarhülse 1 und entsprechendem Überdruck in der Bauchhöhle entsteht auch in dem taschenförmigen Bereich 17 ein entsprechender Überdruck, der auf die Außenseite des kuppelförmigen Wandabschnittes 5 drückt. Aufgrund der gewählten Kuppelform werden durch diesen Überdruck die Dichtlippen 8 verstärkt zusammengepresst, so dass eine automatische Abdichtung des Ventils 3 erfolgt, wenn ein Instrument aus der Trokarhülse entnommen wird. Aufgrund der gewählten Form wirken die durch den Überdruck auf die Dichtlippen 8 einwirkenden Kräfte in tangentialer Richtung, wie es durch zwei Pfeile 18 angedeutet ist, so dass eine optimale Abdichtung der Dichtlippen 8 erreicht wird.

Auch aus den Figuren 7 und 8 ist der genaue Aufbau des Ventils 3 nochmals näher ersichtlich, wobei aus Figur 8 insbesondere die einstückige Ausbildung der Verstärkungsstege 13 mit dem kuppelförmigen Wandabschnitt 5 zu erkennen ist. Grundsätzlich können zusätzlich oder anstelle der innen liegenden Verstärkungsstege 13 auch entsprechende außen liegende Verstärkungsstege vorgesehen sein.

### Bezugszeichenliste

- 1: Trokarhülse
- 2: Ventilgehäuse
- 3: Ventil
- 4: Einführkanal
- 5: kuppelförmiger Wandabschnitt
- 6: schlitzförmige Öffnung
- 7: distaler Scheitelpunkt
- 8: Dichtlippen
- 9: Basisabschnitt
- 10: Flansch
- 11: Einführbereich
- 12: Ventilklappen
- 13: Verstärkungsstege
- 14: Instrument
- 15: Ansätze
- 16: Begrenzungswand
- 17: taschenförmiger Bereich
- 18: Pfeile

## Patentansprüche

1. Trokarhülse mit einem Ventilgehäuse (2) und mit einem in dem Ventilgehäuse (2) angeordneten Ventil (3), das elastisch aneinander anliegende Dichtlippen (8) sowie einen sich in distaler Richtung verengenden, durch einen Wandabschnitt (5) des Ventils (3) gebildeten Einführbereich (11) für ein einzuführendes Instrument (14) besitzt, wobei die Dichtlippen (8) von einem durch den Einführbereich (11) hindurch geführten Instrument (14) auseinander gedrückt werden, der den Einführbereich (11) bildende Wandabschnitt (5) des Ventils (3) zumindest in seinem distalen Bereich kuppelförmig ausgebildet ist, zur Bildung der Dichtlippen (8) in dem kuppelförmigen Wandabschnitt (5) zumindest eine schlitzförmige Öffnung (6) vorgesehen ist, sich die Dichtlippen (8) von dem distalen Endpunkt (7) des Einführbereichs (11) in proximaler Richtung entlang des kuppelförmigen Wandabschnitts (5) erstrecken und zwischen der Außenseite des kuppelförmigen Wandabschnitts (5) und einer diesen Wandabschnitt umfassenden-Begrenzungswand (16) zumindest ein taschenförmiger Bereich (17) ausgebildet ist
**dadurch gekennzeichnet,**
**dass** sich die schlitzförmigen Öffnungen (6) im distalen Endpunkt (7) des kuppelförmigen Wandabschnitts (5) schneiden, **dass** der kuppelförmige Wandabschnitt (5) durch die schlitzförmigen Öffnungen (6) in vier im Wesentlichen gleich große, symmetrisch zum Schnittpunkt der schlitzförmigen Öffnungen (6) angeordneten Ventilklappen (12) aufgeteilt ist, dass jede Ventilklappe (12) mit zumindest einem als Versteifungssteg (13) ausgebildeten Versteifungselement versehen ist, und dass die Versteifungsstege (13) ausgehend von dem proximalen Ende der Ventilklappe (12) in Richtung des distalen Endes der Ventilklappe (12)verlaufen und sich in distaler Richtung verjüngend ausgebildet sind.

2. Trokarhülse nach Anspruch 1,
**dadurch gekennzeichnet,**
**dass** der kuppelförmige Wandabschnitt (5) die Form einer Kugelkalotte besitzt.

3. Trokarhülse nach Anspruch 1 oder 2,
**dadurch gekennzeichnet ,**
**dass** zumindest der den Einführbereich (11) bildende kuppelförmige Wandabschnitt (5) aus elastischem Material, insbesondere aus Kunststoff, ausgebildet ist.

4. Trokarhülse nach einem der vorhergehenden Ansprüche,
**dadurch gekennzeichnet,**
**dass** die schlitzförmigen Öffnungen (6) in einer Draufsicht auf den kuppelförmigen Wandabschnitt (5) Geraden bilden.

5. Trokarhülse nach einem der vorhergehenden Ansprüche,
**dadurch gekennzeichnet,**
**dass** die Versteifungselemente (13) einstückig mit den Ventiklappen (12) ausgebildet sind.

6. Trokarhülse nach einem der vorhergehenden Ansprüche,
**dadurch gekennzeichnet,**
**dass** die Versteifungselemente (13) aus dem gleichen Material wie die Ventilklappen (12) oder aus einem anderen Material als die Ventilklappen (12), jedoch ebenfalls elastisch, ausgebildet sind.

7. Trokarhülse nach einem der vorhergehenden Ansprüche,
**dadurch gekennzeichnet,**
**dass** die Versteifungselemente (13) an der innen liegenden Seite der Ventilklappen (12) vorgesehen sind.

8. Trokarhülse nach einem der vorhergehenden Ansprüche,
**dadurch gekennzeichnet,**
**dass** am proximalen Ende des Einführbereichs (11) ein insbesondere ringförmiger Basisabschnitt (9) des Ventils (3) ausgebildet ist.

9. Trokarhülse nach Anspruch 8,
**dadurch gekennzeichnet,**
**dass** der ringförmige Basisabschnitt (9) einen sich in distaler Richtung erstreckenden ringförmigen Flansch (10) umfasst.

10. Trokarhülse nach einem der vorhergehenden Ansprüche,
**dadurch gekennzeichnet,**
**dass** die Begrenzungswand (16) als Teil des Ventilgehäuses (2) oder als separates Teil ausgebildet ist.

11. Trokarhülse nach einem der Ansprüche 1 bis 9,
**dadurch gekennzeichnet,**
**dass** die Begrenzungswand (16) als Teil des Ventils (3) ausgebildet ist.

12. Trokarhülse nach einem der vorhergehenden Ansprüche,
**dadurch gekennzeichnet,**
**dass** die Begrenzungswand (16) luftdicht mit dem proximalen Ende des Ventils (3) verbunden ist.

13. Trokarhülse nach einem der vorhergehenden Ansprüche,
**dadurch gekennzeinet,**
dass die Begrenzungswand (16) luftdicht mit dem Basisabschnitt (9) des Ventils (3) verbunden ist.

14. Trokarhülse nach einem der vorhergehenden Ansprüche,
**dadurch gekennzeichnet,**
**dass** die Begrenzungswand (16) im Bereich der proximalen Enden der Ventilklappen (12) luftdicht mit dem Ventil (3) verbunden ist.

15. Trokarhülse nach einem der vorhergehenden Ansprüche,
**dadurch gekennzeichnet,**
**dass** das Ventil (3) einstückig, insbesondere als einstückiges Kunststoffteil ausgebildet ist.

16. Trokarhülse nach einem der vorhergehenden Ansprüche,
**dadurch gekennzeichnet,**
**dass** der taschenförmige Bereich (17) den kuppelförmigen Wandabschnitt (5) ringförmig umgibt.

## Claims

1. A trocar sleeve having a valve housing (2) and having a valve (3) which is arranged in the valve housing (2) and which has elastically mutually contacting sealing lips (8) as well as an introduction region (11) narrowing in the distal direction and formed by a wall section (5) of the valve (3) for an instrument (14) to be introduced, wherein the sealing lips (8) are pressed apart by an instrument (14) guided through the introduction region (11), wherein the wall section (5) of the valve (3) forming the introduction region (11) is made dome-shaped at least in its distal region, wherein at least one slot-like opening (6) is provided in the dome-shaped wall section (5) for the formation of the sealing lips (8), wherein the sealing lips (8) extend from the distal end point (7) of the introduction region (11) in the proximal direction along the dome-shaped wall section (5) and wherein at least one pocket-like region (17) is formed between the outer side of the dome-shaped wall section (5) and a boundary wall (16) including this wall section,
**characterised in that**
the slot-like openings (6) intersect at the distal end point (7) of the dome-shaped wall section (5);
**in that** the dome-shaped wall section (5) is subdivided by the slot-shaped openings (6) into four valve flaps (12) which are of substantially the same size and are arranged symmetrically to the point of intersection (7) of the slot-shaped openings (6);
**in that** each valve flap (12) is provided with at least one stiffening element formed as a stiffening web (13); and
**in that** the stiffening webs (13) extend in the direction of the distal end of the valve flap (12), starting from the proximal end of the valve
flap (12), and converge in the distal direction.

2. A trocar sleeve in accordance with claim 1,
**characterised in that**
the dome-shaped wall section (5) has the shape of a spherical cap.

3. A trocar sleeve in accordance with claim 1 or claim 2,
**characterised in that**
at least the dome-shaped wall section (5) forming the introduction region (11) is made of an elastic material, in particular of plastic.

4. A trocar sleeve in accordance with any one of the preceding claims,
**characterised in that**
the slot-shaped openings (6) form straight lines in a plan view of the dome-shaped wall section (5).

5. A trocar sleeve in accordance with any one of the preceding claims,
**characterised in that**
the stiffening elements (13) are made in one piece with the valve flaps (12).

6. A trocar sleeve in accordance with any one of the preceding claims,
**characterised in that**
the stiffening elements (13) are made of the same material as the valve flaps (12) or of a different material to the valve flap (12), but likewise elastically.

7. A trocar sleeve in accordance with any one of the preceding claims,
**characterised in that**
the stiffening elements (13) are provided at the inwardly disposed
side of the valve flaps (12).

8. A trocar sleeve in accordance with any one of the preceding claims,
**characterised in that**
an in particular ring-shaped base section (9) of the valve (3) is formed at the proximal end of the introduction region (11).

9. A trocar sleeve in accordance with claim 8,
**characterised in that**
the ring-shaped base section (9) includes a ring-shaped flange (10) extending in the distal direction.

10. A trocar sleeve in accordance with any one of the preceding claims,
**characterised in that**
the boundary wall (16) is made as part of the valve housing (2) or as a separate part.

11. A trocar sleeve in accordance with any one of the claims 1 to 9,
**characterised in that**
the boundary wall (16) is made as a part of the valve (3).

12. A trocar sleeve in accordance with any one of the preceding claims,
**characterised in that**
the boundary wall (16) is connected to the proximal end of the valve (3) in an airtight manner.

13. A trocar sleeve in accordance with any one of the preceding claims,
**characterised in that**
the boundary wall (16) is connected to the base section (9) of the
valve (3) in an airtight manner.

14. A trocar sleeve in accordance with any one of the preceding claims,
**characterised in that**
the boundary wall (16) is connected to the valve (3) in an airtight manner in the region of the proximal ends of the valve flaps (12).

15. A trocar sleeve in accordance with any one of the preceding claims,
**characterised in that**
the valve (3) is made in one piece, in particular as a one-piece plastic part.

16. A trocar sleeve in accordance with any one of the preceding claims,
**characterised in that**
the pocket-like region (17) surrounds the dome-shaped wall section (5) in a ring shape.

## Revendications

1. Manchon de trocart comportant un boîtier de soupape (2) et une soupape (3) agencée dans le boîtier de soupape (2), laquelle possède des lèvres d'étanchéité (8) en appui élastique l'une sur l'autre ainsi qu'une zone d'introduction (11) pour un instrument (14) à introduire, formée par un tronçon de paroi (5) de la soupape (3) et se rétrécissant en direction distale, les lèvres d'étanchéité (8) étant pressées pour être écartées l'une de l'autre par un instrument (14) guidé à travers la zone d'introduction (11), le tronçon de paroi (5) formant la zone d'introduction (11), de la soupape (3) étant réalisé en forme de coupole au moins dans sa région distale, au moins une ouverture (6) en forme de fente étant prévue dans le tronçon de paroi (5) en forme de coupole pour former les lèvres d'étanchéité (8), les lèvres d'étanchéité (8) s'étendant depuis le point d'extrémité (7) distal de la zone d'introduction (11) en direction proximale le long du tronçon de paroi (5) en forme de coupole, et au moins une région (17) en forme de poche étant réalisée entre la face extérieure du tronçon de paroi (5) en forme de coupole et une paroi de limitation (16) comprenant ce tronçon de paroi,
**caractérisé en ce que**
les ouvertures (6) en forme de fentes se coupent au point d'extrémité distale (7) du tronçon de paroi (5) en forme de coupole, **en ce que** le tronçon de paroi (5) en forme de coupole est divisé en quatre clapets de soupape (12) sensiblement de même taille, agencés symétriquement par rapport au point d'intersection des ouvertures (6) en forme de fentes, **en ce que** chaque clapet de soupape (12) est pourvu d'au moins un élément de raidissement réalisé sous forme de barrette de raidissement (13), et **en ce que** les barrettes de raidissement (13) s'étendent en partant de l'extrémité proximale du clapet de soupape (12) en direction de l'extrémité distale du clapet de soupape (12) et sont formées en se rétrécissant en direction distale.

2. Manchon de trocart selon la revendication 1,
**caractérisé en ce que**
le tronçon de paroi (5) en forme de coupole possède la forme d'une calotte sphérique.

3. Manchon de trocart selon la revendication 1 ou 2,
**caractérisé en ce que**
au moins le tronçon de paroi (5) en forme de coupole formant la zone d'introduction (11) est réalisé en matériau élastique, en particulier en matière plastique.

4. Manchon de trocart selon l'une quelconque des revendications précédentes,
**caractérisé en ce que**
les ouvertures (6) en forme de fentes forment des droites en vue de dessus sur le tronçon de paroi (5) en forme de coupole.

5. Manchon de trocart selon l'une quelconque des revendications précédentes,
**caractérisé en ce que**
les éléments de raidissement (13) sont réalisés d'un seul tenant avec les clapets de soupape (12).

6. Manchon de trocart selon l'une quelconque des revendications précédentes,
**caractérisé en ce que**
les éléments de raidissement (13) sont réalisés dans le même matériau que les clapets de soupape (12) ou en un autre matériau que les clapets de soupape (12), toutefois également élastique.

7. Manchon de trocart selon l'une quelconque des revendications précédentes,
**caractérisé en ce que**
les éléments de raidissement (13) sont prévus sur le côté situé à l'intérieur des clapets de soupape (12).

8. Manchon de trocart selon l'une quelconque des revendications précédentes,
**caractérisé en ce que**
à l'extrémité proximale de la zone d'introduction (11) est réalisé un tronçon de base (9) en particulier de forme annulaire, de la soupape (3).

9. Manchon de trocart selon la revendication 8,
**caractérisé en ce que**
le tronçon de base (9) de forme annulaire comprend une bride (10) de forme annulaire s'étendant en direction distale.

10. Manchon de trocart selon l'une quelconque des revendications précédentes,
**caractérisé en ce que**
la paroi de limitation (16) est réalisée en tant que partie du boîtier de soupape (2) ou en tant que partie séparée.

11. Manchon de trocart selon l'une quelconque des revendications 1 à 9,
**caractérisé en ce que**
la paroi de limitation (16) est réalisée en tant que partie de la soupape (3).

12. Manchon de trocart selon l'une quelconque des revendications précédentes,
**caractérisé en ce que**
la paroi de limitation (16) est reliée de façon hermétique à l'air avec l'extrémité proximale de la soupape (3).

13. Manchon de trocart selon l'une quelconque des revendications précédentes,
**caractérisé en ce que**
la paroi de limitation (16) est reliée de façon hermétique à l'air avec le tronçon de base (9) de la soupape (3).

14. Manchon de trocart selon l'une quelconque des revendications précédentes,
**caractérisé en ce que**
la paroi de limitation (16) est reliée de façon hermétique à l'air avec la soupape (3) dans la région des extrémités proximales des clapets de soupape (12).

15. Manchon de trocart selon l'une quelconque des revendications précédentes,
**caractérisé en ce que**
la soupape (3) est réalisée d'un seul tenant, en particulier en tant que pièce de matière plastique d'un seul tenant.

16. Manchon de trocart selon l'une quelconque des revendications précédentes,
**caractérisé en ce que**
la région (17) en forme de poche entoure à la manière d'un anneau le tronçon de paroi (5) en forme de coupole
